# EUROPÄISCHE PATENTSCHRIFT

(11) **EP 0 456 668 B1**
(45) Veröffentlichungstag und Bekanntmachung des Hinweises auf die Patenterteilung: **19.01.1994**
(21) Anmeldenummer: 90902151.1
(22) Anmeldetag: 03.02.1990
(51) Int. Cl.: A61B 5/091

(54) **EXSPIRATIONSLUFT-AUFNAHMEGEFÄSS**
VESSEL FOR RECEIVING EXHALED AIR
RESERVOIR POUR RECEPTION D'AIR EXPIRE

(30) Priorität: 04.02.1989 DE 3903370
(43) Veröffentlichungstag der Anmeldung: 21.11.1991
(73) Patentinhaber: GANSHORN MEDIZIN ELEKTRONIK GMBH, D-97618 Niederlauer (DE)
(72) Erfinder: GANSHORN, Peter, D-8732 Münnerstadt (DE)
(74) Vertreter: Pöhner, Wilfried Anton, Dr.
(86) Internationale Anmeldenummer: DE9000075
(87) Internationale Veröffentlichungsnummer: WO9008504

(56) Entgegenhaltungen:
- FR-A- 630 982
- US-A- 3 082 761
- US-A- 3 669 097
- US-A- 3 875 626
- Journal of Applied Physiology, vol. 19, 1964, Seiten 534-535; M.A. Sackner et al.: "A bag-in-box system for maintaining gases at body temperature within the body plethysmograph"

## Beschreibung

Die Erfindung betrifft ein Exspirationsluft-Aufnahmegefäß zur Messung der Lungenfunktion, bei dem sich in einem geschlossenen Behälter einander gegenüberliegend zwei Einlässe befinden, die alternierend mit Exspirationsluft beaufschlagbar sind und innerhalb des Behälters ein in Richtung auf die Einlässe verschiebbarer Kolben oder ein Beutel angeordnet ist.

Zur Ermittlung der Leistungsfähigkeit und/oder Erkrankungen der Lunge ist es bekannt, Volumen und Zusammensetzung der ausgeatmeten Luft eines Probanden in Abhängigkeit sich ändernder körperlicher Belastungen gleichzeitig zu erfassen und zu analysieren. Von besonderem Interesse ist der jeweilige Anteil an Sauerstoff und Kohlendioxyd. Die Konzentration der ausgeatmeten Luft unterliegt bereits durch Änderungen der Belastung Schwankungen, die möglichst schnell erfaßt und angezeigt werden sollen. Diese Anforderungen erfüllen Massenspektrometer, die aber in Anschaffung und Unterhalt enorm aufwendig und kostspielig sind. Aus diesem Grunde wird im Augenblick die Exspirationsluft einem Beutel zugeführt, der einen Auslaß aufweist, so daß ein Teil der darin befindlichen Luft austreten und eine Überlastung mit Sicherheit ausgeschlossen werden kann. Ein Teil der angesammelten Luft wird entnommen und in ihrer Zusammensetzung analysiert. Nachteilig ist, daß bei sich ändernder Konzentration der ausgeatmeten Luft durch den sofortigen Austritts eines Teils und das große Volumen sich die Zusammensetzung des Beutels nur langsam ändert. Die Zeitkonstante zur Messung von Konzentrationsänderungen der dem Beutel entnommenen Luft mit Hilfe eines Gasanalysators ist deshalb beträchtlich. Daneben wäre es wünschenswert, wenn auch das Ausatemvolumen bestimmbar wäre.
Vorrichtungen gattungsgemäßer Art sind aus der FR-A-630 982 bekannt, aus der sich ein Gerät zur Durchführung von Atemübungen entnehmen läßt, welches aus einem Behälter besteht, in dessen Inneren ein in axiale Richtung geführte, verschiebbare Zwischenwand oder ein Kolben angeordnet ist, der vermittels eines Einlasses einseitig beaufschlagbar ist. Durch die Zuordnung einer Skala läßt sich in Abhängigkeit von der erfolgten Verschiebung ein definiertes Atemvolumen bestimmen. Ein selbsttätiges Umschalten mit Erreichen des maximalen Volumens ist naturgemäß nicht vorgesehen.

Hiervon ausgehend hat sich die Erfindung die Schaffung eines Exspirationsluft-Aufnahmegefäßes zur Aufgabe gemacht, das eine kurze Ansprechzeit besitzt, einfach im Aufbau ist und darüberhinaus die Messung des Ausatemvolumens ermöglicht.

Erfindungsgemäß wird diese Aufgabe dadurch gelöst, daß der jeweils nicht beaufschlagte Einlaß mit der Atmosphäre in Verbindung steht sowie Schalter vorhanden sind, die mit Erreichen der stirnseitigen Wandung des Behälters durch den Kolben betätigt werden und hierdurch ein Wechsel des beaufschlagten Einlasses eintritt.

Die Benutzung erfolgt in der üblichen Weise, d.h., daß die Exspirationsluft des Probanden vom Mundstück zum Einlaß geführt wird und in das Innere des geschlossenen Behälters eintritt. Gemäß dem Gedanken der Erfindung ist stets nur ein einziger der Einlässe beaufschlagt. Hierdurch entsteht in dem durch Kolben, den entsprechenden Wandungen des Behälters und dem momentan beaufschlagten Einlaß umgrenzten Raum ein Überdruck, durch den der Kolben auf den gegenüberliegenden Einlaß zu bewegt wird, wo er mit Erreichen der entsprechenden, diese umgebenden stirnseitigen Wandung einen Schalter betätigt. Hierdurch erfolgt ein Wechsel in der Beaufschlagung der beiden Einlässe, d.h., daß die von nun an zugeführte Exspirationsluft den Kolben in die Gegenrichtung bewegt und die dort befindliche Luft nach außen preßt. Der nicht beaufschlagte Einlaß führt in die Atmosphäre und der Kolben ist möglichst reibungsfrei zu lagern, damit der von der ausgeatmeten Luft zu überwindende Widerstand gering bleibt und beim Ausatmen natürliche Verhältnisse vorliegen. Durch die Geometrie des Behältnisses wird ein exakt bestimmbares Volumen und damit Atemvolumen definiert. Die Dimensionierung wird idR so bemessen sein, daß mehrere Ausatemprozesse das Gesamtvolumen erfüllen. Es bildet sich ein Mittelwert der Gaskonzentration über mehrere Ausatemprozesse. Wenn durch Bewegungsumkehr des Kolbens das bis zu diesem Zeitpunkt aufgebaute Volumen entleert wird, kann ein Teil des dort befindlichen Gasgemisches entnommen und einem Gasanalysator zugeführt werden. Man mißt den Mittelwert über mehrere Ausatemvorgänge.
Als entscheidender Vorteil ist anzusehen, daß einmal des gemessene Volumen stets konstant ist und Änderungen der Zusammensetzungen der Atemluft relativ rasch erfaßt und analysiert werden können, da mit Umkehrung der Bewegungsrichtung des Kolbens ein Gasvolumen völlig neuer Konzentrationen aufgebaut, über mehrere Atemvorgänge gemittelt und anschließend der Analyse zugeführt wird. Einer der Kerngedanken vorliegender Erfindung besteht darin, die Verwendung relativ einfacher und preisgünstiger Analysegeräte, andererseits jedoch eine möglichst rasche Angleichung und Verfolgung von Änderungen der Gaskonzentration zu gestatten. Im Vergleich zum Stande der Technik erfordert die erfindungsgemäße Vorrichtung keine aufwendigen Analysegeräte und gestattet trotzdem ein demgegenüber wesentlich schnelleres Erfassen und Verfolgen von Änderungen der Atemluftkonzentration.

Unabhängig von dem bisher beschriebenen Exspirations-Aufnahmegefäß wird eine weitere Lösung der erfindungsgemäßen Aufgabe vorgeschlagen, die sich dadurch kennzeichnet, daß der jeweils nicht beaufschlagte Einlaß mit der Atmosphäre in Verbindung steht und innerhalb des Behälters zwei aneinander anliegende Beutel, die über jeweils einen Einlaß beaufschlagbar sind und sich hierbei aufeinander zu bewegen, angeordnet sind sowie ein Schalter, der bei vollständigem Ausfüllen des Innenraumes des Behälters durch den beaufschlagten Beutel betätigt wird und den beaufschlagten Einlaß wechselt.

Die soeben definierte Vorrichtung ist mit der vorbeschriebenen in funktioneller Hinsicht weitgehend ähnlich, unterscheidet sich jedoch durch den im Inneren des Behälters befindlichen Aufbau. Anstelle des darin befindlichen verschiebbaren Kolbens werden zwei aneinander anliegende Beutel untergebracht, die jeweils über einen der Einlässe beaufschlagbar sind.

Bei Beaufschlagung vergrößert sich das Volumen des einen Beutels und bewirkt, daß die im anderen Beutel befindliche Luft nach außen gepreßt wird. Erst, wenn der gesamte Beutel das Innere des Behälters - abzüglich des Eigenvolumens der Wandung des ande ren Beutels - ausfüllt, erhält man ein Volumen definiert. Durch Wechsel der Beaufschlagung vergrößert sich das Volumen des gegenüberliegenden Beutels und die Luft wird aus dem ersteren nach außen abgegeben. In funktioneller Hinsicht übernehmen die aneinanderliegenden Wandungen der Beutel die Funktion des sich in vorbeschriebener Ausführungsform hin und her bewegenden Kolbens. Eine der entscheidenden Vorteile demgegenüber besteht in der Möglichkeit einer demgegenüber kostengünstigeren Herstellung. Die Wandungen des Behälters müssen, um die ungehinderte Bewegung des Beutels zu gestatten, mit Öffnungen versehen sein.

Im übrigen besteht weitgehende Analogie hinsichtlich der Funktionsweise und auch der damit erzielbaren Vorteile mit der erstgenannten Lösung.

Die bisher beschriebenen einzig Zwei Einlässe aufweisenden Exspirationsluft-Aufnahmegefäße müssen zwingend Vorkehrungen aufweisen, die bewirken, daß der augenblicklich nicht beaufschlagte Einlaß mit der Atmosphäre in Verbindung tritt, so daß die Luft entweichen kann. Der Einsatz entsprechender Ventile ist diesbezüglich denkbar, hat jedoch die Schaffung eines zusätzlichen Strömungswiderstandes zur Folge, der in nachteiliger Weise den zum Bewegen von Beutel/Kolben erforderlichen Druck und damit den Ausatemwiderstand erhöht. Eine mögliche bauliche Lösung besteht in der zusätzlichen Anbringung eines der entweichenden Luft dargebotenen Auslasses mit großem Querschnitt. Ebenso und unabhängig davon kann eine der Zurverfügungstellung von Gasproben oder dem Anschluß an einen Gasanalysator dienende Entnahmeöffnung angebracht sein. Eine mögliche weitere Öffnung kann zur Rückführung des entnommenen Gasvolumens genutzt werden.

Der Wechsel der Beaufschlagung der beiden Einlässe in der durch die Schalter gesteuerten Weise wird bevorzugt dadurch erfolgen, daß sich der vom Mundstück ausgehende Schlauch in einen mit einem 2-Wege Ventil versehenen Punkt verzweigt und von dort jeweils einem Einlaß zugeführt ist. Gesteuert über die Schalter wird das 2-Wege Ventil stets einen einzigen der Einlässe mit Exspirationsluft beaufschlagen.

Als Schalter können mechanische oder elektromechanische Konstruktionen eingesetzt werden. Im Hinblick auf die Leichtgängigkeit und der Vermeidung möglicher Widerstände empfiehlt sich der Einsatz von ein berührungsloses Betätigen des Schalters ermöglichenden Lichtschranken. Sie können jedoch auch als Indikatoren für bestimmte (diskrete) Positionen von Kolben oder Beutel genutzt werden.

In vorteilhafter Ausgestaltung wird ein Differenzdruckwandler als Schalter eingesetzt, der den Druck in beiden durch die Beutel oder den Kolben gebildeten Kammern gleichzeitig erfaßt und den entsprechenden Schalter betätigt, wodurch der Wechsel der Beaufschlagung eintritt.

Bei Bewegen in einer nicht mit der Horizontalen zusammenfallenden Richtung bewirkt eine Komponente der Schwerkraft eine abwärts gerichtete Beschleunigung. Damit hierdurch keine Verfälschung des Meßergebnisses auftritt, müssen besondere, einen Ausgleich bewirkende Vorkehrungen getroffen werden. Zu deren Vermeidung und zur Gewährleistung eines möglichst einfachen und damit preisgünstigen Aufbaues ist deshalb von Vorteil, die Bewegung des Kolbens oder Beutels in der Horizontalen auszurichten.

Nach einer gewissen Dauer der Benutzung läßt sich die Ansammlung von in der Atemluft befindlichen Feuchtigkeit innerhalb des Aufnahmegefäßes nicht vermeiden. Zu deren Aufnahme und auch Beseitigung ist auf der Unterseite, also dort, wo sich die Feuchtigkeit vornehmlich ansammelt, die Anbringung einer Wasserfalle von Vorteil.

Weitere Einzelheiten, Merkmale und Vorteile der Erfindung lassen sich dem nachfolgenden Beschreibungsteil entnehmen, indem anhand der Zeichnung ein Ausführungsbeispiel der Erfindung näher erläutert wird. Es zeigen:

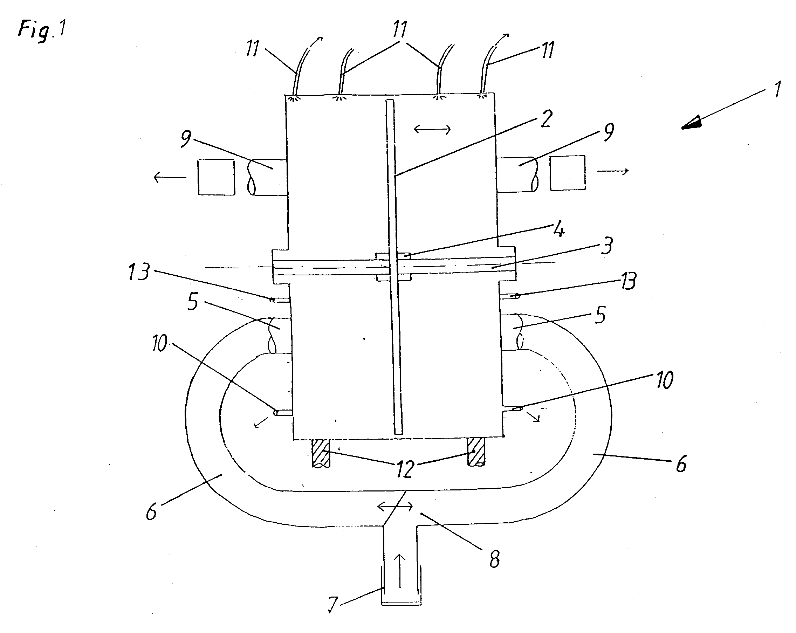

- Figur 1: eine Ausführungsform mit im Behälter bewegbaren Kolben
- Figur 2: einen Behälter mit zwei darin angeordneten Beuteln
In einem Behälter (1) befindet sich ein Kolben (2), der auf einer mittig angeordneten Schiene (3) mit Hilfe eines Lagers (4) aufgesetzt ist. Um mit Erreichen des Umkehrpunktes das Restvolumen zu Null zu machen, ist die Seitenwand entsprechend der Kontur von Kolben (2) mit Lager (4) geformt.

Am Behälter (1) ist sowohl rechts als auch links ein Einlaß (5) vorgesehen, an dem jeweils ein Schlauch (6) endet, der von einem Mundstück (7) ausgeht, sich in einem mit einem 2-Wege-Ventil (8) versehenen Punkt verzweigt und von dort bis zu den beiden Einlässen (5) geführt ist. Die über das Mundstück (7) aufgenommene Exspirationsluft wird durch das 2-Wege Ventil (8) einem der Einlässe (5) zugeführt.

Zusätzlich findet sich im Behälter (1) jeweils auf der Seite der Einlässe (5) ein dem Entweichen der Luft dienender Auslaß (9), durch den die zu entweichende Luft nach außen abgegeben wird, sowie eine der Entnahme von Gasproben dienenden Entnahmeöffnung (10), sowie eine Rückführungsöffnung (13). Hieran kann unmittelbar ein Gasanalysator angeschlossen sein.

Das 2-Wege Ventil (8) wird durch Schalter (11) betätigt und folglich die Bewegung des Kolbens (2) bestimmt.

Auf der Unterseite befinden sich zwei der Aufnahme der in der Atemluft befindlichen Feuchtigkeit dienende Wasserfallen (12).

Die Funktion ist wie folgt: Der Proband gibt über das Mundstück (7) Exspirationsluft ein, die bestimmt durch das 2-Wege Ventil (8) einem der Einlässe (5) zugeführt wird. Von dort gelangt sie in das Innere des vom Kolben (2) und den entsprechenden Wandungen des Behälters (1) umgrenzten Raums, wobei der Auslaß (9) und ebenso die Entnahmeöffnung (10) geschlossen ist, so daß sich ein über dem in der gegenüberliegenden Kammer herrschender Druck aufbaut, so daß sich der Kolben (2) in Richtung auf die dem beaufschlagten Einlaß (5) gegenüberliegenden Wandung des Behälters (1) zu bewegt. Die dort angesammelte Luft wird über den geöffneten Auslaß (9) nach außen abgegeben. Über die zugehörige Entnahmeöffnung (10) werden zu analysierende Gasproben entnommen.
Mit Erreichen der stirnseitigen Wandung wird der Schalter (11) betätigt, das 2-Wege Ventil (8) geschaltet und der bisher unbeaufschlagte Einlaß (5) mit Exspirationsluft versorgt, der dieser Seite zugeordnete Auslaß (9) und evtl. die Entnahmeöffnung (10) geschlossen und der gegenüberliegende Auslaß (9) öffnet, so daß sich im Ergebnis der Kolben (2) in die entgegengesetzte Richtung bewegt. Der oben beschriebene Vorgang kehrt sich um.

Sinn und Zweck der erfindungsgemäßen Vorrichtung ist auf möglichst einfache Art und Weise einen Mittelungsprozeß über mehrere Ausatemvorgänge vorZunehmen, hieraus eine Gasprobe zur Verfügung zu stellen, zu deren Analyse preiswerte Geräte mit vergleichsweise hoher Zeitkonstante verwend- und einsetzbar sind. Andererseits soll sichergestellt sein, daß Änderungen der Gaskonzentration hinreichend rasch, d.h. innerhalb einer gewißen, durch das Volumen des Aufnahmegefäßes bestimmten Zeit exakt erfaßt und erfolgt werden können.

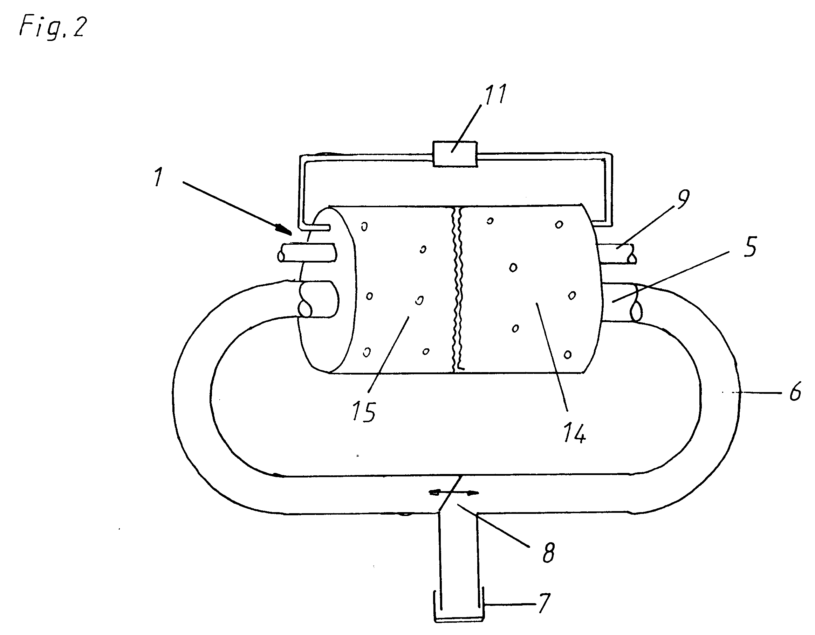

Figur 2 zeigt ebenfalls einen Behälter (1), in dessen Inneren jedoch anstelle des Kolbens jeweils zwei Beutel (14), (15) angebracht sind, deren gegenseitige Zuordnung so erfolgt, daß sie mit ihrem jeweiligen Boden aneinander anzuliegen kommen. Über jeweils einen Einlaß (5) wird einer der beiden Beutel (14), (15) alternierend beaufschlagt. Des weiteren findet sich ein mit einem Ventil versehener Auslaß (9).
Mit Beaufschlagung eines Beutels vergrößert sich dessen Volumen unter gleichzeitiger Verdrängung des anderen Beutels so lange bis das Innere des Behälters vollständig ausgefüllt ist. Hierdurch wird das erfaßte Volumen definiert. Anschließend wird auf den anderen Beutel umgeschaltet und der Vorgang beginnt seitenvertauscht von neuem. Die Änderung des Volumens des Beutels geschieht durch Zusammenfalten und/oder elastisches Ausdehnen der Wandung.

Die Einstellung der Beaufschlagung geschieht mit dem oben bereits beschriebenen 2-Wege-Ventil (8), dessen Steuerung über einen Differenzdruckwandler erfolgt. Hierzu wird aus dem Inneren jedes der Beutel (14), (15) der Druck abgegriffen, miteinander verglichen und zur Schaltung des Ventiles (8) genutzt.

Der Behälter (1) ist mit Öffnungen versehen, um die ungestörte Entfaltung der Beutel (14), (15) im Inneren zu garantieren.

## Patentansprüche

1. Exspirationsluft-Aufnahmegefäß zur Messung der Lungenfunktion, bei dem sich in einem geschlossenen Behälter (1) einander gegenüberliegend zwei Einlässe (5) befinden, die alternierend mit Exspirationsluft beaufschlagbar sind und innerhalb des Behälters (1) ein in Richtung auf die Einlässe (5) verschiebbarer Kolben (2) angeordnet ist, **dadurch gekennzeichnet,** daß der jeweils nicht beaufschlagte Einlaß (5) mit der Atmosphäre in Verbindung steht sowie Schalter (11) vorhanden sind, die mit Erreichen der stirnseitigen Wandung des Behälters (1) durch den Kolben (2) betätigt werden und hierdurch ein Wechsel des beaufschlagten Einlasses (5) eintritt.

2. Exspirationsluft-Aufnahmegefäß zur Messung der Lungenfunktion, bei dem sich in einem Behälter (1) zwei Einlässe (5) befinden, die alternierend mit Exspirationsluft beaufschlagbar sind sowie im Behälter (1) ein Beutel (14,15) angeordnet ist, **dadurch gekennzeichnet,** daß der jeweils nicht beaufschlagte Einlaß (5) mit der Atmosphäre in Verbindung steht und innerhalb des Behälters (1) zwei aneinander anliegende Beutel (14,15), die über jeweils einen Einlaß (5) beaufschlagbar sind und sich hierbei aufeinander zu bewegen, angeordnet sind sowie ein Schalter (11), der bei vollständigem Ausfüllen des Innenraumes des Behälters (1) durch den beaufschlagten Beutel (14,15) betätigt wird und den beaufschlagten Einlaß (5) wechselt.

3. Gefäß nach Anspruch 1 oder 2, **dadurch gekennzeichnet,** daß sich neben dem Einlaß (5) ein Auslaß (9) und/oder eine Entnahmeöffnung (10) sowie eine Öffnung (13) zur Rückführung des entnommenen Gasvolumens befindet.

4. Gefäß nach Anspruch 3, **dadurch gekennzeichnet,** daß die Entnahmeöffnung (10) und die Rückführungsöffnung (13) an einen Gasanalysator angeschlossen sind.

5. Gefäß nach einem der Ansprüche 1 bis 4, **dadurch gekennzeichnet,** daß das Mundstück (7) über ein 2-Wege Ventil (8) mit dem zu den Einlässen (5) führenden Schlauch (6) verbunden ist.

6. Gefäß nach einem der Ansprüche 1 bis 5, **gekennzeichnet durch** Lichtschranken als Schalter (11), und als Indikator diskreter Positionen von Kolben (2) oder Beutel (14, 15).

7. Gefäß nach einem der Ansprüche 1 bis 5, **dadurch gekennzeichnet,** daß der Schalter (11) ein den Druck in beiden Kammern erfassender Differenzdruckwandler ist.

8. Gefäß nach einem der Ansprüche 1 bis 7, **gekennzeichnet durch** horizontale Bewegung von Kolben (2) oder Beutel (14, 15).

9. Gefäß nach einem der Ansprüche 1 bis 8, **gekennzeichnet durch** eine an der Unterseite angeordnete Wasserfalle (12).

## Claims

1. Expiratory air reception vessel for the measurement of lung function, in which in a closed container (1) there are disposed two opposing inlets (5) which can be impinged alternately with expiratory air and within container (1) a piston (2) is arranged which can be shifted toward inlets (5), **wherein** the respective non-impinged inlet (5) is connected with the atmosphere and switches (11) exist which are actuated when said piston (2) reaches the front wall of said container (1), thus causing the impinged inlet (5) to change.

2. Expiratory air reception vessel for the measurement of lung function, in which in a closed container (1) there are disposed two opposing inlets (5) which can be impinged alternately with expiratory air and in container (1) a bag (14, 15) is arranged, **wherein** the respective non-impinged inlet (5) is connected with the atmosphere and within said container (1) are disposed said two bags (14, 15) adjacent to each other, which can be impinged via one of said inlets (5) and which thereby move toward each other, and a switch (11), which is actuated by the impinged bag (14, 15) when the interior space of said container (1) is completely filled, changing the impinged inlet (5).

3. Vessel according to claim 1 or 2, **wherein** an outlet (9) and/or extraction opening (10) as well as an opening (13) for the return of the a removed gas volume are disposed next to said inlet (5).

4. Vessel according to claim 3 **wherein** said extraction opening (10) and said return opening (13) are connected to a gas analyzer.

5. Vessel according to one of the claims 1 to 4 **wherein** mouth piece (7) is connected via 2-way valve (8) to a tube (6), which leads to said inlets (5).

6. Vessel according to one of the claims 1 to 5 **wherein** photoelectric barriers are said switches (11) and indicators of discrete positions of said piston (2) or said bags (14, 15).

7. Vessel according to one of the claims 1 to 5 **wherein** said switch (11) is a differential-pressure transformer, which records the pressure in both chambers.

8. Vessel according to one of the claims 1 to 7 **wherein** said piston (2) or said bags (14, 15) move horizontally.

9. Vessel according to one of the claims 1 to 8 **wherein** a water trap (12) is disposed on the bottom side.

## Revendications

1. Réservoir pour réception d'air expiré pour la mesure de la fonction pulmonaire, dans lequel deux orifices d'entrée (5) dans une enceinte fermée (1), qui peuvent être soumis en alternance à l'air expiré, sont placés l'un en face de l'autre, et un piston (2) déplaçable dans la direction des orifices d'entrée (5) étant disposé à l'intérieur de l'enceinte (1), **caractérisé en ce que** l'orifice d'entrée (5) qui n'est pas activé est ouvert à l'atmosphère et que des interrupteurs (11) sont prévus, qui sont actionnés lorsque le piston (2) atteint la paroi extrême de l'enceinte (1), de sorte qu'une permutation de l'orifice d'entrée activé (5) se produit.

2. Réservoir pour réception d'air expiré pour la mesure de la fonction pulmonaire, dans lequel deux orifices d'entrée (5) qui peuvent être soumis en alternance à l'air expiré sont placés dans une enceinte (1), et une poche (14, 15) étant disposée dans l'enceinte (1), **caractérisé en ce que** l'orifice d'entrée (5) qui n'est pas activé est ouvert à l'atmosphère et qu'à l'intérieur de l'enceinte (1) sont disposées deux poches (14, 15) en contact l'une contre l'autre, chacune pouvant être activée à travers un orifice d'entrée (5) respectif et se déplacer alors l'une en direction de l'autre, ainsi qu'un interrupteur (11), qui lorsque l'espace intérieur de l'enceinte (1) est complètement rempli par la poche (14, 15) est actionné qui est activée et permute l'orifice d'entrée qui est activé.

3. Réservoir selon la revendication 1 ou 2, **caractérisé en ce que** à côté de l'orifice d'entrée (5) se trouve un orifice de sortie (9) et/ou une ouverture de prélèvement (10) ainsi qu'une ouverture (13) pour la réinjection du volume de gaz prélevé.

4. Réservoir selon la revendication 3, **caractérisé en ce que** l'ouverture de prélèvement (10) et l'ouverture de réinjection (13) sont reliées à un analyseur de gaz.

5. Réservoir selon l'une des revendications 1 à 4, **caractérisé en ce que** l'embout buccal (7) est relié par un tuyau (6) conduisant aux orifices d'entrée (5) à travers une soupape bistable (8).

6. Réservoir selon l'une des revendications 1 à 5, **caractérisé par** des barrages photoélectriques, comme interrupteurs (11), et comme indicateurs d'un nombre discret de positions du piston (2) ou des poches (14, 15).

7. Réservoir selon l'une des revendications 1 à 5, **caractérisé en ce que** l'interrupteur (11) est un capteur de pression différentielle, qui détecte la pression dans chacune des deux chambres.

8. Réservoir selon l'une des revendications 1 à 7, **caractérisé par** un mouvement du piston (2) ou des poches (14, 15) suivant une direction horizontale.

9. Réservoir selon l'une des revendications 1 à 8, **caractérisé par** un piège à eau (12) situé à la surface inférieure.
